# FASCICULE DE BREVET EUROPEEN

(11) **EP 2 542 518 B1**
(45) Date de publication et mention de la délivrance du brevet: **07.05.2014**
(21) Numéro de dépôt: 11712969.2
(22) Date de dépôt: 04.03.2011
(51) Int. Cl.: C07C 51/29, C07C 51/31, C07C 67/313, C07C 67/31, C07C 67/333, C07C 51/377

(54) **PROCEDE DE PREPARATION D'ACIDES CARBOXYLIQUES PAR COUPURE OXYDANTE D'UN DIOL VICINAL**
PROCESS ZUR HERSTELLUNG VON CARBONSÄUREN DURCH OXIDATIVE SPALTUNG VON VICINALEN DIOLEN
PROCESS FOR PREPARING CARBOXYLIC ACIDS BY OXIDATIVE CLEAVAGE OF A VICINAL DIOLS

(30) Priorité: 05.03.2010 FR 1051627
(43) Date de publication de la demande: 09.01.2013
(73) Titulaire: Organisation Nationale Interprofessionnelle des Graines et Fruits Oleagineux (O.N.I.D.O.L.), 75008 Paris (FR); Centre National de la Recherche Scientifique, 75016 Paris (FR); Université Claude Bernard Lyon I, 69622 Villeurbanne Cedex (FR)
(72) Inventeur: LEMAIRE, Marc, F-69100 Villeurbanne (FR); FAVRE-REGUILLON, Alain, F-01090 Montmerle Sur Saone (FR); PAQUIT, Bénédicte, F-42300 Roanne (FR); CLAUDE, Sylvain, F-75013 Paris (FR); RAOUL, Yann, F-02650 Crezancy (FR)
(74) Mandataire: Hubert, Philippe
(86) Numéro de dépôt international: PCT/FR2011/050456
(87) Numéro de publication internationale: WO 2011/107721

(56) Documents cités:
- FR-A5- 2 086 521
- DATABASE WPI Week 200949 Thomson Scientific, London, GB; AN 2009-L49077 XP002615356, & JP 2009 155320 A (LION CORP) 16 juillet 2009 (2009-07-16)

## Description

La présente invention a pour objet un nouveau procédé de préparation d'acides carboxyliques, en particulier d'acides mono- et di-carboxyliques, par coupure oxydante d'un diol vicinal.

L'invention trouve notamment application pour la valorisation des huiles végétales d'origine naturelle, en particulier les huiles extraites d'oléagineux, ainsi que des huiles animales riches en acides gras polyinsaturés.

On sait que les huiles végétales et les produits qui en dérivent, tels que les esters, les acides gras sont couramment utilisés en l'état ou en tant qu'intermédiaires pour la synthèse de nombreux produits de spécialité.

Les huiles végétales constituent une source de matières renouvelables abondamment disponibles et particulièrement intéressantes dans la mesure où les acides gras qui les composent sont riches en acide oléique. En effet, les produits issus de la transformation de cet acide insaturé par coupure oxydante, à savoir l'acide pélargonique et l'acide azélaïque, sont utilisés comme précurseurs d'esters destinés à la lubrification et à la plastification ainsi qu'en tant qu'intermédiaires pour la production de polymères.

Cette réaction de coupure oxydante de l'acide oléique peut être représentée par le schéma réactionnel suivant :

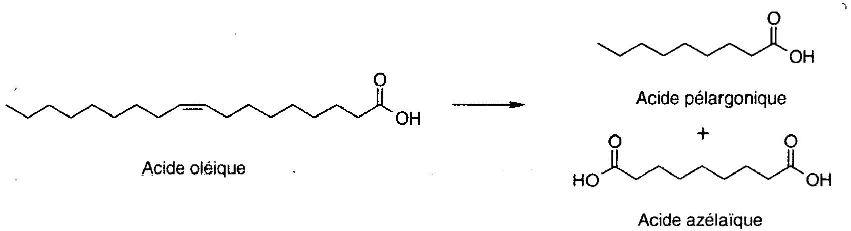

On connaît déjà divers procédés permettant d'obtenir les acides pélargonique et azélaïque par coupure oxydante de l'acide oléique.

Ainsi, ces acides peuvent être obtenus directement par ozonolyse de l'acide oléique (voir revue Rebrovic et coll. Lipids Technology (1996), 135-137). Ce procédé est actuellement mis en oeuvre à l'échelle industrielle. Cependant, l'utilisation d'ozone est particulièrement délicate en raison de sa dangerosité.

Des procédés catalytiques de clivage oxydant de l'acide oléique utilisant des métaux de transition et divers oxydants (Re₂O₇/H₂O₂, H₂WO₄/H₂O₂, Ru(acac)_{3/}NaIO₄, RuO₂/NaOCl, RuCl₃/MeCO₃H) ont été proposés dans la littérature. Cependant, ces procédés n'ont pas fait l'objet d'une exploitation industrielle car ils ne sont pas suffisamment compétitifs pour concurrencer l'ozonolyse.

Des procédés alternatifs de préparation en deux étapes peuvent également être envisagés.

Un premier procédé connu consiste, dans une première étape, à préparer l'acide 9-décènoïque par une réaction de métathèse sur l'acide oléique, puis dans une deuxième étape à procéder à la coupure oxydante de la double liaison terminale de cet acide. Cette coupure oxydante est plus facile à réaliser avec les systèmes catalytiques précédents.

Dans le cadre de ce procédé en deux étapes, dont le schéma réactionnel est représenté ci-dessous, on obtient l'acide azélaïque et le 1-décène en tant que sous-produits.

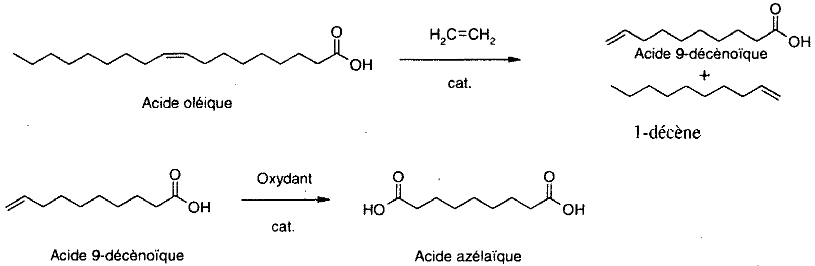

Les inventeurs ont exploré un autre procédé consistant :
- dans une première étape, à préparer un diol de l'acide oléique selon les méthodes décrites dans la littérature, par exemple par oxydation catalytique en utilisant l'eau oxygénée et un catalyseur à base de tungstène ou de rhénium ou un système acide carboxylique/H₂O₂ comme oxydant ; puis
- dans une seconde étape, à réaliser la coupure oxydante de ce diol pour former les acides pélargonique et azélaïque.

Ce procédé peut être représenté par le schéma réactionnel suivant :

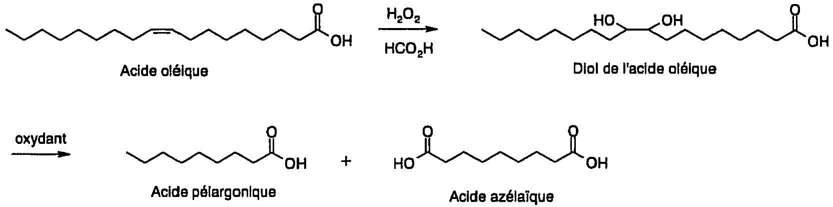

Selon une variante de ce procédé, l'acide pélargonique et l'acide azélaïque peuvent être obtenus par ouverture de l'époxyde de l'acide oléique, par la mise en oeuvre des mêmes conditions réactionnelles.

Cette variante peut être représentée par le schéma réactionnel suivant : 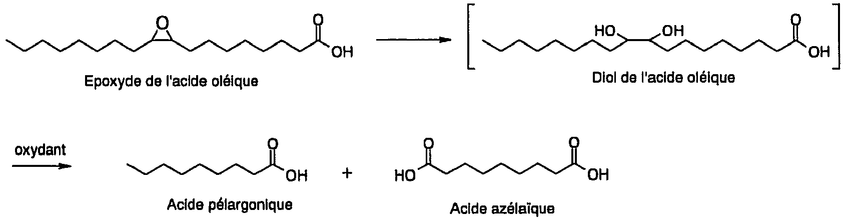 Dans le brevet Français N°2 086 521, il est proposé de réaliser la coupure oxydante de divers diols vicinaux, dont le diol de l'acide oléique, avec du monopersulfate de potassium et une quantité catalytique de nickel (20 mol. %).

Dans le document JP 2009 155320, il est proposé de préparer des acides carboxyliques à partir de l'acide oléique par coupure oxydante, à l'aide d'hyprochlorite de sodium.

Dans la littérature, divers procédés ont également été proposés pour réaliser la coupure de certains diols utilisant des catalyseurs à base de métaux de transition et divers oxydants (Re₂O₇/H₂O₂, RuCl₃/H₂O₂, NiCl₂/NaOCl, ...) souvent en milieu organique. Cependant ces procédés sont rarement employés pour la coupure du diol de l'acide oléique ou d'un diol analogue. En outre, l'utilisation de catalyseurs à base de métaux de transition est généralement à éviter, voire même interdite, à l'échelle industrielle car ces produits, et en particulier le nickel, sont particulièrement toxiques.

Il a été découvert, et ceci constitue le fondement de la présente invention, qu'il était possible de réaliser la coupure oxydante de diols variés, en particulier de diols d'acides carboxyliques insaturés, tels que notamment l'acide oléique , ou de leurs dérivés, comme par exemple, les esters d'acides gras , d'une façon particulièrement simple, peu coûteuse, respectueuse de l'environnement et ne posant aucun problème de sécurité.

Ainsi selon un premier aspect, la présente invention a pour objet un procédé de préparation d'acides carboxyliques, en particulier d'acides mono- et di-carboxyliques, par coupure oxydante d'un diol vicinal, caractérisé en ce qu'il consiste à faire réagir un diol vicinal de formule I : 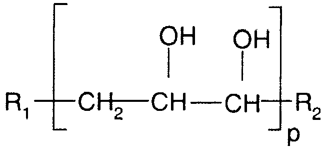 dans laquelle :
p est un nombre entier compris entre 1 et 6, de préférence égal à 1 ou 2;
R₁ et R₂ représentent indépendamment :
   - un groupe alkyle ou hydroxyalkyle ayant de 1 à 12 atomes de carbone ;
   - un groupe -(CH₂)ₙ-CO₂M dans lequel n, qui peut être identique ou différent dans R₁ et R₂, est un nombre entier compris entre 1 et 11 et M représente un atome d'hydrogène ou un groupe alkyle ayant de 1 à 4 atomes de carbone ou un cation alcalin ;
ou R₁ et R₂ forment ensemble un groupe alkylène -(CH₂)ₘ- dans lequel m est un nombre entier compris entre 2 et 10, de préférence entre 2 et 6 ;
avec de l'hypochlorite de sodium (ou javel) de qualité industrielle, en l'absence de solvant organique et sans ajout de catalyseur, de préférence à température ambiante.

Comme on le comprend, l'originalité du procédé conforme à l'invention réside dans le fait qu'il utilise un oxydant facilement disponible, de faible coût et dont la mise en oeuvre est réalisée dans des conditions de grande sécurité et respectueuses de l'environnement car en l'absence de solvant organique et surtout sans ajout de catalyseur et de préférence à basse température.

Dans la présente demande, on entend désigner par "hypochlorite de sodium de qualité industrielle", les produits commerciaux habituellement disponibles dans l'industrie présentant une quantité comprise entre 10 et 20% en chlore actif.

Par "groupe alkyle", on entend désigner ici une chaîne hydrocarbonée saturée linéaire ou ramifiée, de préférence linéaire.

Par "groupe hydroxyalkyle", on entend désigner ici un groupe alkyle dont l'un au moins des atomes d'hydrogène est remplacé par un groupe hydroxy.

Selon une caractéristique particulière de l'invention, le diol précité répond à la formule (I) dans laquelle :
R₁ et R₂ représentent indépendamment :
   - un groupe alkyle ayant de 5 à 9 atomes de carbone ;
   - un groupe -(CH₂)ₙ-CO₂M dans lequel n, qui peut être identique ou différent dans R₁ et R₂, est un nombre entier compris entre 5 et 9 et M représente un atome d'hydrogène ou un groupe alkyle ayant de 1 à 4 atomes de carbone ; et p est de préférence égal à 1.

Le procédé conforme à l'invention est généralement utile pour la coupure oxydante de diols dérivés d'acides mono-insaturés ou poly-insaturés et de leurs dérivés, tels que, par exemple, les esters d'acides gras correspondants ; en particulier à chaîne longue (ayant notamment plus de 10 atomes de carbone, de préférence de 10 à 30 atomes de carbones), provenant de préférence de source naturelle et en particulier des oléagineux, par exemple : huile de soja, huile de tournesol, huile de colza, huile de lin, huile d'olive, huile de ricin, huile d'arachide, huile de palme, etc.

A titre d'exemple d'acide mono-insaturé, on peut citer l'acide myristoléique (acide 9-tétradécènoïque), l'acide palmitoléique (acide 9-hexadécènoïque), l'acide oléique (acide 9-octadécènoïque), l'acide ricinoléique (acide 12-hydroxy-9-octadécènoïque), l'acide gadoléique (acide 11-eicosènoïque), l'acide érucique (acide 13-docosènoïque), l'acide nervonique (acide 15-tétracosènoïque).

A titre d'exemple d'acide poly-insaturé, on peut citer l'acide linoléique (acide 9,12-octadécadiènoïque), l'acide alpha-linolénique (acide 9,12,15-octadécatriènoïque), l'acide gamma-linolénique (acide 6,9,12-octadécatriènoïque), l'acide di-homo-gamma-linolénique (acide 8,11,14-eicosatriènoïque), l'acide arachidonique (acide 5,8,11,14-eicosatétraènoique), l'acide timnodonique (acide 5,8,11,14,17-eicosapentaènoïque), l'acide cervonique (acide 4,7,10,13,16,19-docosahexaénoïque).

Ce procédé est particulièrement approprié pour la coupure oxydante du diol de l'acide oléique (composé de formule I précitée dans laquelle p est égal à 1, R₁ représente un groupe -(CH₂)₆-CH₃ et R₂ représente un groupe -(CH₂)₇COOH) en acide pélargonique et en acide azélaïque.

Ce procédé est également utile pour la coupure oxydante de diols dérivés d'alcènes cycliques et en particulier du diol du cyclohexène qui permet de préparer l'acide adipique dont l'utilisation industrielle pour la fabrication du Nylon est bien connue.

Lorsque l'un des substituants R₁ et R₂ représente un groupe -(CH₂)ₙ-CO₂M (M représentant un atome d'hydrogène ou un groupe alkyle ayant de 1 à 4 atomes de carbones et n, un nombre entier compris entre 1 et 11, de préférence entre 5 et 9) et l'autre représente un groupe alkyle ayant de 1 à 12 atomes de carbone, de préférence de 5 à 9 atomes de carbone, la coupure oxydante du composé de formule (I) conduit à un mélange d'acide mono-carboxylique et d'acide di-carboxylique, comme un mélange d'acide pélargonique et d'acide azélaïque dans le cas de l'acide oléique ou conduit à un mélange d'acide mono-carboxylique et d'hemiester, comme un mélange d'acide pélargonique et d'azélate de mono-alkyle dans le cas d'un oléate d'alkyle.

Lorsque les groupes R₁ et R₂ représentent simultanément un groupe de formule -(CH₂)ₙ-CO₂M (n pouvant être différent dans chacun des substituants R₁ et R₂) la coupure oxydante conduit à un mélange d'acides di-carboxyliques voire même à un acide di-carboxylique unique lorsque le diol de départ est symétrique, c'est-à-dire lorsque R₁ représente un groupe -(CH₂)ₙ₋₁-CO₂M et R₂ représente un groupe -(CH₂)ₙ-CO₂M.

Ainsi, selon une caractéristique particulièrement avantageuse de l'invention, le diol de départ répond à la formule (I) dans laquelle :
R₁ représente :
   - un groupe -(CH₂)ₙ₋₁-CO₂M dans lequel n est un nombre entier compris entre 6 et 9 et M représente un atome d'hydrogène ou un cation alcalin ;
R₂ représente :
   - un groupe -(CH₂)ₙ-CO₂M dans lequel n , qui est identique dans R₁ et R₂, est un nombre entier compris entre 6 et 9 et M représente un atome d'hydrogène ou un cation alcalin; et p est de préférence égal à 1.

Dans le cas particulier où le diol de départ est l'acide 9,10-dihydroxy-octadécanedioïque, il est possible d'obtenir l'acide azélaïque exempt de sous-produit, tel que l'acide pélargonique.

D'une façon tout à fait surprenante, il a été montré dans le cadre de la présente invention que les diols vicinaux obtenus à partir d'acides gras insaturés ou poly-insaturés peuvent être isolés par recristallisation ou par extraction avec un degré de pureté particulièrement élevé, supérieur à 98 %, à partir d'une source naturelle de ces acides.

Par exemple, l'acide 9,10-dihydroxy-octadécanedioïque peut être obtenu, à partir de l'acide oléique, par un procédé en deux étapes comprenant :
- dans un premier temps, la conversion de l'acide oléique, en acide 9-octadécènedioïque, notamment par la mise en oeuvre du procédé de bioconversion décrit dans la demande de brevet internationale WO 2006/064131, puis
- dans une deuxième étape, à procéder à la dihydroxylation de l'acide 9-octadécènedioïque précité notamment à l'aide d'un mélange d'eau oxygénée et d'un acide organique comme l'acide formique ou l'acide acétique, comme il sera décrit plus en détail ci-dessous.

Selon une caractéristique particulièrement avantageuse de l'invention, l'acide 9-octadécènedioïque est obtenu par bioconversion dans laquelle on soumet une souche mutante de Yarrowia lipolytica à un substrat de bioconversion consistant en une huile de tournesol oléique.

Il a été observé, d'une façon tout à fait inattendue, que ce procédé en deux étapes permet d'obtenir un produit présentant une pureté élevée (supérieure à 98 %) même lorsque le produit est de départ (l'acide 9-octadécènedioïque) est obtenu à l'issue de la première étape avec une pureté inférieure (par exemple inférieure à 80 %., voire même de l'ordre de 65 %).

La deuxième étape de ce procédé peut donc être mise en oeuvre au départ d'un acide 9-octadécènedioïque non pur, tel que par exemple un produit commercial d'une pureté de 65 % à 99 %.

La synthèse de l'acide azélaïque à partir d'acide oléique selon la voie qui vient d'être décrite peut être représentée par le schéma réactionnel suivant :

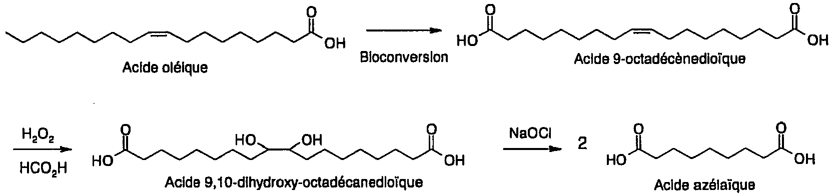

Selon une caractéristique particulière du procédé conforme à l'invention, le diol de formule (I) et l'hypochlorite de sodium sont présents au sein du milieu réactionnel permettant la coupure oxydante dans un rapport molaire de l'hypochlorite de sodium au diol compris entre 2 et 30, de préférence entre 3 et 5.

Selon un mode de réalisation actuellement préféré, le diol de formule (I) précitée est obtenu par Dihydroxylation d'un alcène de formule (II) : 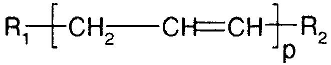
dans laquelle p, R₁ et R₂ sont tels que définis précédemment.

L'alcène de formule (II) est en particulier un acide mono-insaturé ou un acide poly-insaturé, de préférence à chaîne longue, tel que défini précédemment.

Selon une autre caractéristique particulière de l'invention, la dihydroxylation est réalisée à l'aide d'un mélange d'eau oxygénée et d'un acide organique de formule RCO₂H dans laquelle R représente un atome d'hydrogène ou un groupe alkyle ayant de 1 à 4 atomes de carbone, de préférence dans un rapport molaire de l'eau oxygénée à l'acide organique compris entre 1:5 et 1:20.

Avantageusement, la dihydroxylation précitée est réalisée à l'aide d'un mélange d'eau oxygénée et d'acide formique ou d'un mélange d'eau oxygénée et d'acide acétique.

Selon une autre caractéristique particulière de l'invention, l'étape de dihydroxylation est généralement conduite à une température comprise entre 30 et 50°C pendant une durée de 6 à 12 h, par exemple à 40°C pendant une durée de 8 h.

La dihydroxylation permet l'obtention d'un produit dihydroxylé pur à partir d'une coupe d'acide gras d'origine naturelle

L'invention sera mieux comprise à la lecture des exemples illustratifs suivants.

### Exemple 1 : Dihydroxylation du cyclohexène

A un mélange de 6,8 mL d'eau oxygénée à 30% v/v (68,2 mmol, 1,4 eq.) et de 24 mL d'acide formique (0,67 mol, 13,7 eq.) refroidit à 0°C, 4 g de cyclohexène (48,7 mmol) ont été ajoutés gouttes à gouttes. A la fin de l'addition, le mélange a été chauffé à 40°C pendant 8 h puis laissé à température ambiante pendant la nuit. L'eau et l'acide formique ont ensuite été partiellement éliminés sous pression réduite jusqu'à l'obtention d'une huile. L'huile a été reprise dans 50 mL de potasse à 1 N et chauffée à 90°C pendant 1 h. A cette solution, de l'acide chlorhydrique concentré (37 % pds) a été ajouté jusqu'à l'obtention d'un pH voisin de 2. Le cis-1,2-cyclohexanediol a été obtenu par décantation puis filtration sous la forme d'une poudre blanche avec un rendement de 50% (2,83 g)
Pf = 74-76°C, Analyse IRFT, RMN ¹H et ¹³C dans CDCl₃ et analyse GC/MS conformes à la littérature (pureté>99 %).

### Exemple 2 : Dihydroxylation de l'acide oléique à 90% de pureté

A un mélange de 25 mL d'eau oxygénée à 30% v/v (248 mmol, 1,4 eq.) et de 91,3 mL d'acide formique (2,42 mol, 13,7 eq.) refroidit à 0°C, 50 g d'acide oléique (159 mmol) pur à 90% (pureté déterminée par analyse GC/MS de l'ester méthylique correspondant) ont été ajoutés gouttes à gouttes. A la fin de l'addition, le mélange a été chauffé à 40°C pendant 8 h puis laissé à température ambiante pendant la nuit. L'eau et l'acide formique ont ensuite été partiellement éliminés sous pression réduite jusqu'à l'obtention d'une huile. L'huile a été reprise dans 150 mL de potasse à 1 N et chauffée à 90°C pendant 1 h. A cette solution, de l'acide chlorhydrique concentré (37% pds) a été ajouté jusqu'à l'obtention d'un pH voisin de 2. La phase huileuse obtenue a été séparée à l'aide d'une ampoule de coulée et a été lavée avec 100 mL d'eau. On a ainsi obtenu 59 g d'une huile.

A des fins analytiques, cette huile a été recristallisée dans l'hexane et on a ainsi obtenu l'acide 9,10-dihydroxystéraïque pur sous la forme d'un solide blanc. Pf = 130-132°C, Analyse IRFT, RMN ¹H et ¹³C dans MeOD et analyse GC/MS de l'ester méthylique conformes à la littérature.

### Exemple 3 : Dihydroxylation de l'acide 9-octadécènedioïque à 65 % par le système HCO₂H/H₂O₂/CH₂Cl₂

L'acide 9-octadécènedioïque utilisés dans cet exemple est le produit commercialisé par la société SEDERMA sous la dénomination de O.D.A. (pur à 65 %).

L'acide 9-octadécènedioïque (65 mmol, 31,24 g) pur à 65 % (pureté déterminée par analyse GC/MS de l'ester méthylique correspondant) a été ajouté en 1 h sur un mélange H₂O₂ 30 % v/v (0,14 mol, 14,3 mL) / HCO₂H (1,4 mol, 52,8 mL) / CH₂Cl₂ (50 mL) à 0°C. A la fin de l'addition, le milieu réactionnel a été chauffé à 40°C pendant 8 h puis laissé à température ambiante pendant la nuit. L'eau et l'acide formique ont été partiellement évaporés sous pression réduite jusqu'à obtention d'une huile. L'huile a été reprise dans 100 mL de potasse à 1 N et chauffée à 90°C pendant 1 h. A cette solution, de l'acide chlorhydrique concentré (37 % pds) a été ajouté jusqu'à l'obtention d'un pH acide. L'acide 9,10-dihydroxyoctadécanedioïque précipité a été récupéré sous la forme d'une poudre blanche par simple filtration avec un rendement quantitatif (24,2 g).
Pf = 158-160°C, Analyse IRFT, RMN ¹H et ¹³C dans MeOD et analyse GC/MS de l'ester méthylique conformes à la littérature (pureté>99 %).

### Exemple 4: Dihydroxylation de l'acide 9-octadecènedioïque à 65 % par le système CH₃CO₂H / H₂O₂

L'acide 9-octadécènedioïque (19,5 mmol, 9,37 g) pur à 65 % (pureté déterminée par analyse GC/MS de l'ester méthylique correspondant) a été dissout dans 35 mL d'acide acétique concentré. Cette solution a été ajouté en 30 minutes sur un mélange H₂O₂ 30% v/v (42 mmol, 9,6 mL) / CH₃CO₂H (0,42 mol, 24 mL) à 0°C. A la fin de l'addition, le milieu réactionnel a été chauffé à 40°C pendant 8 h puis laissé à température ambiante pendant la nuit. L'eau et l'acide acétique ont été partiellement évaporés sous pression réduite jusqu'à obtention d'une huile. L'huile a été reprise dans 50 mL de potasse à 1 N et chauffée à 90°C pendant 1 h. A cette solution, de l'acide chlorhydrique concentré (37 % pds) a été ajouté jusqu'à l'obtention d'un pH voisin de 2. L'acide 9,10-dihydroxyoctadécanedioïque précipité a été récupéré sous la forme d'une poudre blanche par simple filtration avec un rendement quantitatif (6,75 g).

### Exemple 5: Dihydroxylation de l'oléate de méthyle à 75 %

L'oléate de méthyle utilisé dans cet exemple est un produit commercialement disponible (grade technique).

L'oléate de méthyle (7,5 mmol, 2,96 g) pur à 75 % (pureté déterminée par analyse GC/MS) a été ajouté en 25 minutes sur un mélange H₂O₂ 30% v/v (14 mmol, 1,43 mL) / HCO₂H (0,14 mol, 5,05 mL) à 0°C. A la fin de l'addition, le milieu réactionnel a été chauffé à 40°C pendant 8 h puis laissé à température ambiante pendant la nuit. L'eau et l'acide formique ont été partiellement évaporés sous pression réduite jusqu'à obtention d'une huile. L'huile a été reprise dans 50 mL de potasse à 1 N et chauffée à 90°C pendant 1 h. A cette solution, de l'acide chlorhydrique concentré (37 % pds) a été ajouté jusqu'à l'obtention d'un pH voisin de 2. La phase huileuse obtenue a été séparée à l'aide d'une ampoule de coulée et a été lavée avec 5 mL d'eau.

On a ainsi obtenu une huile avec un rendement quantitatif (3,3 g).

A des fins analytiques, cette huile a été recristallisée dans l'hexane et le 9,10-dihydroxystéarate de méthyle a été obtenu pur sous la forme d'un solide blanc.
Pf= 158-160°C, Analyse IRFT, RMN ¹H et ¹³C dans MeOD et GC/MS conformes à la littérature.

### Exemple 6: Clivage oxydant du trans-1,2-cyclohexanediol

A 21,6 mL (31,5 mmol) d'une solution de Javel à 1,46 mol.L⁻¹ (Acros Ref 21925) ont été additionnés 1,16 g (10 mmol) de trans-1,2-cyclohexanediol. Après 3 h à température ambiante, la phase aqueuse a été acidifiée avec de l'HCl 37 % poids. L'acide adipique a été obtenu par filtration avec un rendement de 50 % (0.73 g) sous la forme d'un solide blanc.
Pf= 150-152°C, Analyse IRFT, RMN ¹H et ¹³C dans DMSO conformes à la littérature.

### Exemple 7: Clivage oxydant de l'acide 9,10-dihydroxy-octadécanoïque

A 10,8 mL (15,75 mmol) d'une solution de Javel à 1,46 mol.L⁻¹ (Acros Ref 21925) ont été additionnés 1,58 g (5 mmol) d'acide 9,10-dihydroxy-octadécanoïque pur. Après 5 h à température ambiante, la phase aqueuse a été acidifiée avec de l'HCl 37 % poids. La phase huileuse obtenue a été séparée à l'aide d'une ampoule de coulée et a été lavée avec 6 mL d'eau. On a ainsi obtenu 1,73 g d'une huile.

A des fins analytiques, le produit a alors été dissout dans 6 mL de MeOH puis 0,6 mL d'acide sulfurique concentré ont été ajoutés. Le milieu réactionnel a été chauffé à reflux pendant 2 h. Le produit a été repris dans 20 mL d'eau. La phase organique a été extraite avec 3 x 6 mL d'hexane. La phase organique obtenue a été lavée successivement avec 20 mL d'eau, 20 mL de solution de carbonate de sodium et 20 mL d'eau. La phase organique a été séchée sur Na₂SO₄ et le solvant a été évaporé sous pression réduite.. On a ainsi obtenu le mélange d'esters attendu avec un rendement quantitatif (1,94 g). Après évaporation du solvant, le produit a été purifié par chromatographie sur silice (cyclohexane/EtOAc: 5/2).

Le pélargonate de méthyle (Rf = 0,84) a été obtenu sous la forme d'une huile limpide avec un rendement de 89 % et l'azélate de diméthyle (Rf = 0,47) a été obtenu sous la forme d'une huile limpide avec un rendement de 40 %. Analyse RMN ¹H et ¹³C dans CDCl₃ et analyse GC/MS du pélargonate de méthyle et de l'azélate de diméthyle conformes à la littérature.

### Exemple 8: Clivage oxydant de l'acide 9,10-dihydroxy-octadécanedioïque

A 8,6 mL (9,45 mmol) d'une solution de Javel à 1,11 mol.L⁻¹ (Aldrich Ref 81070) ont été additionnés 1,04 g (3 mmol) d'acide 9,10-dihydroxy-octadécanedioïque pur. Après 5 h à température ambiante, la phase aqueuse a été acidifiée avec de l'HCl 37 % poids. L'acide azélaïque a été obtenu par décantation puis filtration et a été lavé à l'eau froide.

L'acide azélaïque a été obtenu avec un rendement de 50 % sous la forme d'un solide blanc.
Pf = 107°C, Analyse IRFT, RMN ¹H, ¹³C dans CDCl₃ et GC/MS du diester méthylique conformes à la littérature.

### Exemple 9: Clivage oxydant de l'acide 9,10-dihydroxy-octadécanedioïque

L'acide 9,10-dihydroxy-octadécanedioïque pur (2 mmol, 693 mg) a été empâté avec 3 mL d'une solution aqueuse d'azélate de sodium à 15 % wt. Sur cette pâte a été ajouté 5,7 mL (6,3 mmol) d'une solution de Javel à 1,11 mol.L⁻¹ (Aldrich Ref 81070). Après 5 h à température ambiante, la phase aqueuse a été acidifiée avec de l'HCl 37 % poids. L'acide azélaïque a été obtenu par décantation puis filtration et a été lavé à l'eau froide.

L'acide azélaïque a été obtenu avec un rendement de 50% sous la forme d'un solide blanc.

### Exemple 10: Clivage oxydant du 9,10-dihydroxystéarate de méthyle

A 7,5 mL (11 mmol) d'une solution de Javel à 1,46 mol.L⁻¹ (Acros Ref 21925) a été additionné 1,16 g (3,5 mmol) de 9,10-dihydroxystéarate de méthyle pur. Après 3 h à température ambiante, la phase aqueuse a été acidifiée avec de l'HCl 37% (poids). La phase huileuse obtenue a été séparée à l'aide d'une ampoule de coulée et a été lavée avec 4 mL d'eau. On a ainsi obtenu 1,26 g d'huile.

A des fins analytiques, le produit ainsi obtenu ont alors été dissout dans 2,8 mL de MeOH puis 0,28 mL d'acide sulfurique concentré ont été ajoutés. Le milieu réactionnel a été chauffé à reflux pendant 2 h. Le produit a été repris dans 10 mL d'eau. La phase organique a été extraite avec 3 x 5 mL d'acétate d'éthyle. La phase organique obtenue a été lavée successivement avec 10 mL d'eau, 10 mL de solution de carbonate de sodium et 10 mL d'eau. La phase organique a été séchée sur Na₂SO₄ et le solvant a été évaporé sous pression réduite.

On a ainsi obtenu le mélange d'esters attendu avec un rendement quantitatif (1,36 g).
Analyse GC/MS (après estérification selon la méthode précédemment décrite) conformes à la littérature.

Les exemples qui viennent d'être donnés montrent que le procédé conforme à la présente invention permet de préparer des acides carboxyliques variés, en particulier des acides mono- et di-carboxyliques utiles en tant qu'intermédiaires de synthèse pour la production de polymères, comme notamment l'acide azélaïque et l'acide adipique.

Ce procédé est particulièrement avantageux, dans la mesure où il utilise un oxydant facilement disponible, de faible coût et dont la mise en oeuvre peut être réalisée dans des conditions de grande sécurité et respectueuse de l'environnement.

Ce procédé est également avantageux, dans la mesure où les produits de départ peuvent être d'origine naturelle, comme en particulier l'acide oléique obtenu à partir des huiles extraites d'oléagineux.

## Revendications

1. Procédé de préparation d'acides carboxyliques, en particulier d'acides mono et di-carboxyliques, par coupure oxydante d'un diol vicinal, **caractérisé en ce qu'**il consiste à faire réagir un diol vicinal de formule I : 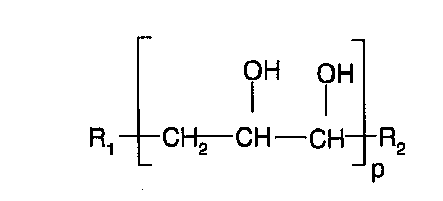 dans laquelle :
p est un nombre entier compris entre 1 et 6;
R₁ et R₂ représentent indépendamment :
- un groupe alkyle ou hydroxyalkyle ayant de 1 à 12 atomes de carbone ;
- un groupe -(CH₂)ₙ-CO₂M dans lequel n, qui peut être identique ou différent dans R₁ et R₂, est un nombre entier compris entre 1 et 11 et M représente un atome d'hydrogène ou un groupe alkyle ayant de 1 à 4 atomes de carbone ou un cation alcalin;
ou R₁ et R₂ forment ensemble un groupe alkylène -(CH₂)ₘ- dans lequel m est un nombre entier compris entre 2 et 10, de préférence entre 2 et 6 ;
avec de l'hypochlorite de sodium (ou javel) de qualité industrielle, en l'absence de solvant organique et sans ajout de catalyseur.

2. Procédé selon la revendication 1, **caractérisé en ce que** la réaction précitée est réalisée à température ambiante.

3. Procédé selon la revendication 1 ou 2, **caractérisé en ce que** le diol précité répond à la formule I dans laquelle:
R₁ et R₂ représentent indépendamment :
- un groupe alkyle ayant de 5 à 9 atomes de carbone ;
- un groupe -(CH₂)ₙ-CO₂M dans lequel n, qui peut être identique ou différent dans R₁ et R₂, est un nombre entier compris entre 5 et 9 et M représente un atome d'hydrogène ou un groupe alkyle ayant de 1 à 4 atomes de carbone ou un cation alcalin.

4. Procédé selon la revendication 1 ou 2, **caractérisé en ce que** le diol précité répond à la formule I dans laquelle:
R₁ représente:
- un groupe -(CH₂)ₙ₋₁-CO₂M dans lequel n est un nombre entier compris entre 6 et 9 et M représente un atome d'hydrogène ou un cation alcalin;
R₂ représente:
- un groupe -(CH₂)ₙ-CO₂M dans lequel n , qui est identique dans R₁ et R₂, est un nombre entier compris entre 6 et 9 et M représente un atome d'hydrogène ou un cation alcalin.

5. Procédé selon la revendication 3 ou 4, **caractérisé en ce que** le diol précité répond à la formule I dans laquelle p est égal à 1.

6. Procédé selon la revendication 4, **caractérisé en ce que** le diol de formule (I) précité est l'acide 9,10-dihydroxy-octadécanedioïque.

7. Procédé selon la revendication 6, **caractérisé en ce que** l'acide 9,10-dihydroxy-octadécanedioïque est obtenu par dihydroxylation de l'acide 9-octadécènedioïque, lui-même obtenu à partir de l'acide oléique.

8. Procédé selon la revendication 6, **caractérisé en ce que** l'acide 9,10-dihydroxy-octadécanedioïque est obtenu par dihydroxylation de l'acide 9-octadécènedioïque, lui-même obtenu à partir de l'acide oléique par bioconversion.

9. Procédé selon l'une des revendications précédentes, **caractérisé en ce que** le diol de formule I et l'hypochlorite de sodium sont mis à réagir dans un rapport molaire de l'hypochlorite de sodium au diol compris entre 3 et 30.

10. Procédé selon l'une des revendications précédentes, **caractérisé en ce que** le diol de formule I et l'hypochlorite de sodium sont mis à réagir dans un rapport molaire de l'hypochlorite de sodium au diol compris entre 3 et 5.

11. Procédé selon l'une des revendications précédentes, **caractérisé en ce que** le diol de formule (I) précité est obtenu par dihydroxylation d'un alcène de formule II : 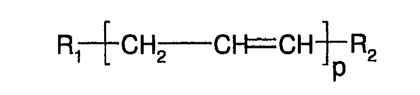
dans laquelle p, R₁ et R₂ sont tels que définis dans ces revendications.

12. Procédé selon la revendication 11, **caractérisé en ce que** la dihydroxylation est réalisée à l'aide d'un mélange d'eau oxygénée et d'un acide organique de formule RCO₂H dans laquelle R représente un atome d'hydrogène ou un groupe alkyle ayant de 1 à 4 atomes de carbone.

13. Procédé selon la revendication 11, **caractérisé en ce que** la dihydroxylation est réalisée à l'aide d'un mélange d'eau oxygénée et d'un acide organique de formule RCO₂H dans laquelle R représente un atome d'hydrogène ou un groupe alkyle ayant de 1 à 4 atomes de carbone, dans un rapport molaire de l'eau oxygénée à l'acide organique compris entre 1 et 20.

14. Procédé selon la revendication 11, **caractérisé en ce que** la dihydroxylation est réalisée à l'aide d'un mélange d'eau oxygénée et d'un acide organique de formule RCO₂H dans laquelle R représente un atome d'hydrogène ou un groupe alkyle ayant de 1 à 4 atomes de carbone, dans un rapport molaire de l'eau oxygénée à l'acide organique compris entre 1 et 5.

15. Procédé selon l'une des revendications 12 à 14, **caractérisé en ce que** la dihydroxylation est réalisée à l'aide d'un mélange d'eau oxygénée et d'acide formique ou d'un mélange d'eau oxygénée et d'acide acétique.

## Patentansprüche

1. Verfahren zur Herstellung von Carbonsäuren, insbesondere von Mono- und Dicarbonsäuren, durch oxidative Spaltung eines vicinalen Diols, **dadurch gekennzeichnet, dass** es darin besteht, ein vicinales Diol der Formel I: 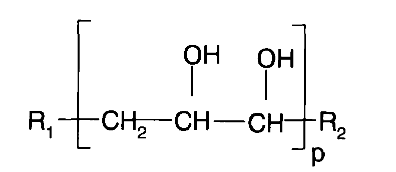 worin:
p eine ganze Zahl zwischen 1 und 6 ist;
R₁ und R₂ unabhängig darstellen:
- eine Alkyl- oder Hydroxyalkyl-Gruppe mit 1 bis 12 Kohlenstoffatomen;
- eine -(CH₂)ₙ-CO₂M-Gruppe, worin n, das bei R₁ und R₂ identisch oder unterschiedlich sein kann, eine ganze Zahl zwischen 1 und 11 ist und M ein Wasserstoffatom oder eine Alkylgruppe mit 1 bis 4 Kohlenstoffatomen oder ein Alkalikation darstellt;
oder R₁ und R₂ zusammen eine -(CH₂)ₘ-Alkylengruppe bilden, worin m eine ganze Zahl zwischen 2 und 10, vorzugsweise zwischen 2 und 6 ist;
mit Natriumhypochlorit (oder Javel) mit Industriequalität in Abwesenheit eines organischen Lösungsmittels und ohne Zugabe eines Katalysators reagieren zu lassen.

2. Verfahren nach Anspruch 1, **dadurch gekennzeichnet, dass** die vorgenannte Reaktion bei Raumtemperatur durchgeführt wird.

3. Verfahren nach Anspruch 1 oder 2, **dadurch gekennzeichnet, dass** das vorgenannte Diol der Formel I entspricht, worin:
R₁ und R₂ unabhängig darstellen:
- eine Alkylgruppe mit 5 bis 9 Kohlenstoffatomen;
- eine -(CH₂)ₙ-CO₂M-Gruppe, worin n, das bei R₁ und R₂ identisch oder unterschiedlich sein kann, eine ganze Zahl zwischen 5 und 9 ist und M ein Wasserstoffatom oder eine Alkylgruppe mit 1 bis 4 Kohlenstoffatomen oder ein Alkalikation darstellt.

4. Verfahren nach Anspruch 1 oder 2, **dadurch gekennzeichnet, dass** das vorgenannte Diol der Formel I entspricht, worin:
R₁ darstellt:
- eine -(CH₂)ₙ₋₁-CO₂M-Gruppe, worin n eine ganze Zahl zwischen 6 und 9 ist und M ein Wasserstoffatom oder ein Alkalikation darstellt;
R₂ darstellt:
- eine -(CH₂)ₙ-CO₂M-Gruppe, worin n, das bei R₁ und R₂ identisch ist, eine ganze Zahl zwischen 6 und 9 ist und M ein Wasserstoffatom oder ein Alkalikation darstellt.

5. Verfahren nach Anspruch 3 oder 4, **dadurch gekennzeichnet, dass** das vorgenannte Diol der Formel I entspricht, worin p gleich 1 ist.

6. Verfahren nach Anspruch 4, **dadurch gekennzeichnet, dass** das vorgenannte Diol der Formel (I) 9,10-Dihydroxy-octadecandisäure ist.

7. Verfahren nach Anspruch 6, **dadurch gekennzeichnet, dass** die 9,10-Dihydroxy-octadecandisäure durch Dihydroxylierung der 9-Octadecendisäure erhalten wird, die selbst aus Ölsäure erhalten wird.

8. Verfahren nach Anspruch 6, **dadurch gekennzeichnet, dass** die 9,10-Dihydroxy-octadecandisäure durch Dihydroxylierung der 9-Octadecendisäure erhalten wird, die selbst aus Ölsäure durch biologische Umwandlung erhalten wird.

9. Verfahren nach einem der vorhergehenden Ansprüche, **dadurch gekennzeichnet, dass** das Diol der Formel I und das Natriumhypochlorit in einem Molverhältnis von Natriumhypochlorit zu Diol zwischen 3 und 30 zur Reaktion gebracht werden.

10. Verfahren nach einem der vorhergehenden Ansprüche, **dadurch gekennzeichnet, dass** das Diol der Formel I und das Natriumhypochlorit in einem Molverhältnis von Natriumhypochlorit zu Diol zwischen 3 und 5 zur Reaktion gebracht werden.

11. Verfahren nach einem der vorhergehenden Ansprüche, **dadurch gekennzeichnet, dass** das vorgenannte Diol der Formel (I) durch Dihydroxylierung eines Alkens der Formel II erhalten wird: 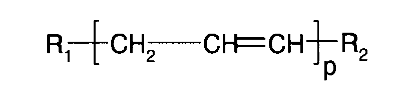
worin p, R₁ und R₂ so sind wie in diesen Ansprüchen definiert.

12. Verfahren nach Anspruch 11, **dadurch gekennzeichnet, dass** die Dihydroxylierung mit Hilfe eines Gemischs aus Wasserstoffperoxid und aus einer organischen Säure der Formel RCO₂H, worin R ein Wasserstoffatom oder eine Alkylgruppe mit 1 bis 4 Kohlenstoffatomen darstellt, vollzogen wird.

13. Verfahren nach Anspruch 11, **dadurch gekennzeichnet, dass** die Dihydroxylierung mit Hilfe eines Gemischs aus Wasserstoffperoxid und aus einer organischen Säure der Formel RCO₂H, worin R ein Wasserstoffatom oder eine Alkylgruppe mit 1 bis 4 Kohlenstoffatomen darstellt, in einem Molverhältnis von Wasserstoffperoxid zu organischer Säure zwischen 1 und 20, vollzogen wird.

14. Verfahren nach Anspruch 11, **dadurch gekennzeichnet, dass** die Dihydroxylierung mit Hilfe eines Gemischs aus Wasserstoffperoxid und aus einer organischen Säure der Formel RCO₂H, worin R ein Wasserstoffatom oder eine Alkylgruppe mit 1 bis 4 Kohlenstoffatomen darstellt, in einem Molverhältnis von Wasserstoffperoxid zu organischer Säure zwischen 1 und 5, vollzogen wird.

15. Verfahren nach einem der Ansprüche 12 bis 14, **dadurch gekennzeichnet, dass** die Dihydroxylierung mit Hilfe eines Gemischs aus Wasserstoffperoxid und aus Ameisensäure oder eines Gemischs aus Wasserstoffperoxid und aus Essigsäure vollzogen wird.

## Claims

1. A method for preparing carboxylic acids, in particular mono- and dicarboxylic acids, by oxidative cleavage of a vicinal diol, **characterized in that** it consists of reacting a vicinal diol of formula I: 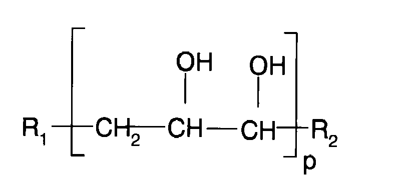 in which:
p is an integer between 1 and 6;
R₁ and R₂ represent independently:
- an alkyl or hydroxyalkyl group having from 1 to 12 carbon atoms;
- a group -(CH₂)ₙ-CO₂M in which n, which can be identical or different in R₁ and R₂, is an integer between 1 and 11 and M represents a hydrogen atom or an alkyl group having from 1 to 4 carbon atoms or an alkaline cation;
or R₁ and R₂ together form an alkylene group -(CH₂)ₘ- in which m is an integer between 2 and 10, preferably between 2 and 6;
with sodium hypochlorite (or bleach) of industrial grade, in the absence of organic solvent and without addition of catalyst.

2. The method as claimed in claim 1, **characterized in that** said a reaction is carried out at room temperature.

3. The method as claimed in claim 1 or 2, **characterized in that** the aforementioned diol corresponds to formula I in which:
R₁ and R₂ represent independently:
- an alkyl group having from 5 to 9 carbon atoms;
- a group -(CH₂)ₙ-CO₂M in which n, which can be identical or different in R₁ and R₂, is an integer between 5 and 9 and M represents a hydrogen atom or an alkyl group having from 1 to 4 carbon atoms or an alkaline cation.

4. The method as claimed in claim 1 or 2, **characterized in that** the aforementioned diol corresponds to formula I in which:
R₁ represents:
- a group -(CH₂)ₙ₋₁-CO₂M in which n is an integer between 6 and 9 and M represents a hydrogen atom or an alkaline cation;
R₂ represents:
- a group -(CH₂)ₙ-CO₂M in which n, which is identical in R₁ and R₂, is an integer between 6 and 9 and M represents a hydrogen atom or an alkaline cation.

5. The method as claimed in claim 3 or 4, **characterized in that** the aforementioned diol corresponds to formula I in which p is equal to 1.

6. The method as claimed in claim 4, **characterized in that** the diol of formula (I) above is 9,10-dihydroxy-octadecanedioic acid.

7. The method as claimed in claim 6, **characterized in that** 9,10-dihydroxy-octadecanedioic acid is obtained by dihydroxylation of 9-octadecenedioic acid, itself obtained from oleic acid.

8. The method as claimed in claim 6, **characterized in that** 9,10-dihydroxy-octadecanedioic acid is obtained by dihydroxylation of 9-octadecenedioic acid, itself obtained from oleic acid, preferably by bioconversion.

9. The method as claimed in one of the preceding claims, **characterized in that** the diol of formula I and sodium hypochlorite are reacted at a molar ratio of sodium hypochlorite to diol between 3 and 30.

10. The method as claimed in one of the preceding claims, **characterized in that** the diol of formula I and sodium hypochlorite are reacted at a molar ratio of sodium hypochlorite to diol between 3 and 5.

11. The method as claimed in one of the preceding claims, **characterized in that** the diol of formula (I) above is obtained by dihydroxylation of an alkene of formula II: 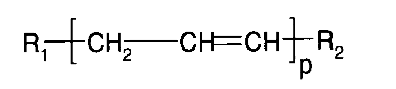
in which p, R₁ and R₂ are as defined in these claims.

12. The method as claimed in claim 11, **characterized in that** dihydroxylation is carried out using a mixture of hydrogen peroxide and an organic acid of formula RCO₂H in which R represents a hydrogen atom or an alkyl group having from 1 to 4 carbon atoms.

13. The method as claimed in claim 11, **characterized in that** dihydroxylation is carried out using a mixture of hydrogen peroxide and an organic acid of formula RCO₂H in which R represents a hydrogen atom or an alkyl group having from 1 to 4 carbon atoms, at a molar ratio of hydrogen peroxide to organic acid between 1 and 20.

14. The method as claimed in claim 11, **characterized in that** dihydroxylation is carried out using a mixture of hydrogen peroxide and an organic acid of formula RCO₂H in which R represents a hydrogen atom or an alkyl group having from 1 to 4 carbon atoms, preferably at a molar ratio of hydrogen peroxide to organic acid between 1 and 5.

15. The method as claimed in any of claims 12 to 14, **characterized in that** dihydroxylation is carried out using a mixture of hydrogen peroxide and formic acid or a mixture of hydrogen peroxide and acetic acid.
